# EUROPÄISCHE PATENTSCHRIFT

(11) **EP 2 170 582 B1**
(45) Veröffentlichungstag und Bekanntmachung des Hinweises auf die Patenterteilung: **26.08.2015**
(21) Anmeldenummer: 08774167.4
(22) Anmeldetag: 20.06.2008
(51) Int. Cl.: B29C 59/16, B81C 1/00, B01L 3/00, G01N 33/52, G01N 33/48, B01J 19/00

(54) **VERFAHREN ZUR HERSTELLUNG EINES MIKROFLUIDEN SYSTEMS AUF EINER POLYMEROBERFLÄCHE**
METHOD FOR MANUFACTURING A MICROFLUID SYSTEM ON A POLYMER SURFACE
PROCÉDÉ DE FABRICATION D'UN SYSTÈME DE MICRO LIQUIDE SUR UNE SURFACE EN POLYMÈRE

(30) Priorität: 03.07.2007 EP 07111621
(43) Veröffentlichungstag der Anmeldung: 07.04.2010
(73) Patentinhaber: Roche Diagnostics GmbH, 68305 Mannheim (DE); F. Hoffmann-La Roche AG, 4070 Basel (CH)
(72) Erfinder: FINKE, Werner, 64683 Einhausen (DE); RÖPER, Josef, K., 67141 Neuhofen (DE); URLAUB, Eva, 55276 Dienheim (DE)
(74) Vertreter: Herzog, Fiesser & Partner Patentanwälte PartG mbB
(86) Internationale Anmeldenummer: PCT/EP2008/057862
(87) Internationale Veröffentlichungsnummer: WO 2009/003856

(56) Entgegenhaltungen:
- WO-A-2006/074665
- DE-A1- 10 224 568
- DE-A1- 19 753 847
- GB-A- 2 350 678
- US-A1- 2003 142 901
- US-A1- 2006 234 269
- US-B1- 6 271 040

## Beschreibung

Die Erfindung bezieht sich auf die Herstellung eines mikrofluiden Systems mit einer Polymeroberfläche, deren Benetzbarkeit in Teilbereichen gezielt modifiziert wird.

Die Mikrofluidik beschäftigt sich mit der Handhabung insbesondere von Flüssigkeiten auf kleinstem Raum. Mikrofluide Systeme sind Bauteile, die dazu dienen, Flüssigkeiten auf Längenskalen unterhalb von 1 mm zu bewegen, zu kontrollieren und zu analysierten. Mikrofluide Systeme werden zum Beispiel für Anwendungen und Messungen in der modernen Biologie, der Biotechnologie, der Biochemie, der pharmazeutischen Industrie, der analytischen und klinischen Chemie, der Umweltanalytik oder der Prozesskontrolle eingesetzt.

Zur Analyse von Körperflüssigkeiten wie Blut oder Urin werden häufig mikrofluide Systeme in Form von Testelementen verwendet. Die zu analysierenden Proben werden auf ein Testelement gegeben und reagieren dort gegebenenfalls mit einer oder mehreren Reagenzien, bevor sie analysiert werden. Die optische, insbesondere die photometrische und die elektrochemische Auswertung von Testelementen stellen gebräuchliche Verfahren zur schnellen Bestimmung der Konzentration von Analyten in der Probe dar. Es gibt verschiedene Formen von Testelementen, beispielsweise Kapillarspalt-Testelemente, bei denen die Probenflüssigkeit von einem Probenaufgabeort zu einem davon entfernten Probendetektionsort mit Hilfe kapillarer Kräfte in einem Transportkanal (Kapillarkanal, Kapillarspalt) bewegt wird, um dort eine Nachweisreaktion einzugehen. Kapillarspalt-Testelemente sind beispielsweise aus CA 2549143 oder aus US 2003/0013147 A1 bekannt. Die Mikrokapillaren tragen eine Innenbeschichtung aus hydrophilen und gegebenenfalls auch aus hydrophoben Materialien. Durch hydrophile und hydrophobe Oberflächeneigenschaften der mit der Probenflüssigkeit in Kontakt tretenden Materialien kann der Flüssigkeitstransport in mikrofluiden Systemen gesteuert werden. Die Funktionalisierung (Hydrophobisierung oder Hydrophilisierung) von Polymeroberflächen erfolgt im Stand der Technik unter anderem durch Beschichten, zum Beispiel aus dem gasförmigen, dampfförmigen, flüssigen, breiigen oder pastenförmigen Zustand, zum Beispiel durch Aufsprühen einer Suspension, aus dem ionisierten Zustand durch elektrolytisches oder chemisches Abscheiden oder aus dem festen Zustand (d.h. körnigem oder pulvrigem Zustand), zum Beispiel durch Pulverbeschichtung oder Beschichtung durch Sintern. Weiterhin ist es zum Beispiel bekannt, Testelemente aus mehreren übereinander liegenden Folien aufzubauen, die verschiedene Benetzbarkeiten aufweisen.

WO 01/56771 A2 beschreibt das Strukturieren einer Polymerschicht durch Plasmaätzen oder Photoablation. Dabei handelt es sich um abtragend wirkende Verfahren, durch die eine dreidimensionale Struktur in miteinander verbundenen Polymerschichten erzeugt wird. Je nachdem, welche der mit verschiedenen Oberflächeneigenschaften ausgestatteten Polymerschichten an eine so erzeugte Struktur angrenzt, ändert sich die Benetzbarkeit der Strukturoberfläche.

WO 98/23957 A1 bezieht sich auf die Erzeugung eines Oberflächenmusters mittels Photoablation. In dem photoablatierten Bereich wird zum Beispiel ein biologisches Affinitätsreagenz aufgetragen.

US 6 271 040 B1 offenbart ein Verfahren gemäß dem Oberbegriff des Anspruchs 1.

Die im Stand der Technik bekannten Verfahren zur Oberflächenfunktionalisierung lassen sich in großflächige und in ortsaufgelöste Methoden einteilen. Großflächige Methoden haben den Nachteil, dass sie gegebenenfalls die Weiterverarbeitung erschweren oder stören. Beispielsweise resultiert das Verkleben einer Schicht mit bestimmten Oberflächeneigenschaften in störenden Klebstoffresten. Abwechselnde Muster von hydrophilen und hydrophoben Funktionen (patterning) sind durch großflächige Methoden nicht herstellbar. Im Stand der Technik bekannte ortsaufgelöste Methoden sind aufwendig und teuer. Die Herstellung kleiner Dimensionen, d.h. einer hohen Auflösung, ist schwierig. Teilweise sind diese ortsaufgelösten Methoden nur auf ebenen Oberflächen anwendbar. Für Änderungen der Geometrie besteht wenig Flexibilität.

Die Aufgabe der Erfindung besteht darin, die Nachteile des Standes der Technik zu vermeiden. Insbesondere ist es Aufgabe der Erfindung, ein Verfahren zur Herstellung eines mikrofluiden Systems auf einer Polymeroberfläche bereitzustellen, bei dem kostengünstig und flexibel die Benetzbarkeit mindestens eines Teilbereichs der Polymeroberfläche modifiziert wird.

Diese Aufgabe wird erfindungsgemäß gelöst durch ein Verfahren zur Herstellung eines mikrofluiden Systems auf einer Polymeroberfläche, gemäß Anspruch 1.

Die Polymeroberfläche kann dabei die Oberfläche eines ebenen Trägers, beispielsweise eines streifen- oder bandförmigen Trägers sein. Es sind mittels des erfindungsgemäßen Verfahrens aber auch Polymeroberflächen modifizierbar, die zu einem beliebig dreidimensional geformten Träger gehören. Der Träger kann mit einem Polymer beschichtet sein oder vollständig aus einem Polymer bestehen. Das Polymer ist vorzugsweise ein Polymer ausgewählt aus der Gruppe bestehend aus Polyethylenterephthalat (Polyester - PET), Polycarbonat (PC), Polyetheretherketon (PEEK), Polystyrol (PS), Polypropylen (PP), Pölymethylmethacrylat (PMMA), Polydimethylsiloxan (PDMS) und cyclisches Olefin-co-Polymer (COC).

Erfindungsgemäß wird mindestens ein Teilbereich der Polymeroberfläche gezielt mit Laserlicht bestrahlt. Gezielt bedeutet in diesem Zusammenhang, dass keine Masken oder ähnliches verwendet werden, sondern dass mindestens ein Laserstrahl mittels geeigneter optischer Komponenten auf den Teilbereich fokussiert wird und diesen Teilbereich abfährt (scannt), so dass eine ortsaufgelöste Modifikation der Polymeroberfläche erreicht wird.

Die Bestrahlung mit Laserlicht wird zum Modifizieren der Benetzbarkeit des Teilbereichs der Polymeroberfläche durchgeführt. Die Benetzbarkeit der Oberfläche (und damit zum Beispiel die Fließgeschwindigkeit in einer mit dieser Oberfläche ausgestatteten Kapillare) lässt sich anhand des Kontaktwinkels α, den Wasser (bzw. eine Wasser enthaltende Probe) mit der Oberfläche bildet, ableiten. Bei der Berührung eines Flüssigkeitstropfens mit einer festen Unterlage können zwei Extremfälle auftreten:
- vollkommene Benetzung: die Adhäsionskräfte sind größer als die Kohäsionskräfte. Deshalb wird sich die Probe auf der Oberfläche des festen Körpers ausbreiten;
- unvollkommene Benetzung: die Adhäsionskräfte sind (wesentlich) kleiner als die Kohäsionskräfte. Deshalb wird sich die Flüssigkeit zu einem kugelförmigen Tropfen zusammenziehen.

Die Benetzbarkeit und folglich zum Beispiel die Fließgeschwindigkeit einer flüssigen Probe in einer Kapillare sind umso größer, je kleiner der Kontaktwinkel α ist. Die Füllzeit zur Füllung einer Kapillare pro Strecke steigt mit dem Kontaktwinkel exponentiell an. Bei Wasser enthaltenden Proben genügt die Angabe des Kontaktwinkels von Wasser, um die materialspezifischen kapillaren Eigenschaften zu charakterisieren. Das erfindungsgemäße mikrofluide System kann sich diesen Effekt zum Beispiel zu Nutze machen, indem die Polymer-Innenoberfläche einer mittels des erfindungsgemäßen Verfahrens behandelten Kapillare in Zonen mit verschiedenen Benetzbarkeiten eingeteilt ist, so dass eine flüssige Probe in diesen Zonen der Kapillare unterschiedliche Kontaktwinkel α bildet und somit kontinuierlich mit sich unterscheidenden Geschwindigkeiten durch diese Zonen der Kapillare fließt. Damit lässt sich gezielt beeinflussen, wie lange sich die Probe in der jeweiligen Zone befindet und zum Beispiel mit dort platzierten Reagenzien reagiert. Folglich können in einer Kapillare eines erfindungsgemäßen mikrofluiden Systems (zum Beispiel eines Testelements) nacheinander verschiedene Messungen durchgeführt werden, insbesondere auch komplexe Messungen, die durch den in Zonen eingeteilten Aufbau der Kapillare und durch die sich daraus ergebende zeitliche Trennung der Reaktionsschritte ermöglicht werden. Bei einer parallelen Anordnung mehrerer Kapillaren in einem Testelement können sogar gleichzeitig und parallel verschiedene Mehrfachmessungen mit einer flüssigen Probe durchgeführt werden.

Die flüssige Probe ist bevorzugt eine Wasser enthaltende Probe, zum Beispiel Plasma, Blut, interstitielle Flüssigkeit, Urin, Proben der Wasseranalytik, insbesondere Abwasser, Speichel oder Schweiß. Vorzugsweise ist das mikrofluide System ein diagnostisches System.

Das erfindungsgemäße Modifizieren der Benetzbarkeit des Teilbereichs der Polymeroberfläche bedeutet, dass mittels des Laserlichts in dem Teilbereich eine Änderung des Kontaktwinkels bewirkt wird, den die flüssige Probe mit der Polymeroberfläche bildet. Das Modifizieren erfolgt jedoch ohne ein Abtragen von Material, insbesondere indem die durch das Laserlicht eingestrahlte Energiedichte unterhalb der Ablationsschwelle liegt.

Ein Vorteil des erfindungsgemäßen Verfahrens besteht darin, dass mittels Laserlicht eine hohe räumliche Auflösung im Hinblick auf den modifizierten Teilbereich erreicht wird. Durch eine geeignete Wahl von Laser und Optik ist eine Ortsauflösung bis in den µm-Bereich möglich.

Gemäß einer bevorzugten Ausführungsform des erfindungsgemäßen Verfahrens wird die Polymeroberfläche in dem mit Laserlicht bestrahlten Teilbereich durch das Bestrahlen mit Laserlicht strukturiert, d.h. die Oberflächenstrukturierung wird durch das Laserlicht verändert. Insbesondere kann die Polymeroberfläche durch das Bestrahlen mit Laserlicht aufgerauht werden. Zur Strukturierung wird ein gepulster Laser verwendet, wobei die Polymeroberfläche in dem Teilbereich mit dem gepulsten Laserstrahl abgespannt wird und durch das Auftreffen der Laserpulse auf die Polymeroberfläche in einem gewissen Abstand zueinander die Polymeroberfläche strukturiert wird. Durch geeignete Wahl der Laserparameter (Wellenlänge, Leistung, Pulsrate, etc.) können gezielt Mikrostrukturen erzeugt werden, die zum Beispiel hydrophile oder hydrophobe Eigenschaften hervorrufen. Durch das Laserlicht entstehen auf der Polymeroberfläche verschmolzene, runde Strukturen (Erhebungen und Vertiefungen), deren mittlerer Abstand (zum Beispiel von Vertiefung zu Vertiefung) mit dem Begriff "Hatch distance" bezeichnet wird.

Durch eine solche Strukturierung können zum Beispiel Teilbereiche der Polymeroberfläche des mikrofluiden Systems so modifiziert werden, dass sie den sogenannten "Lotus-Effekt" aufweisen. Dieser Effekt wird beispielsweise in WO 96/04123 A1, WO 00/58410 A1 oder WO 00/58415 A1 beschrieben. Eine solche Oberfläche weist Erhebungen und Vertiefungen auf, wobei der Abstand zwischen den Erhebungen im Bereich zwischen 0,1 bis 200 µm und die Höhe der Erhebungen im Bereich von 0,1 bis 100 µm liegt, und die Erhebungen hydrophob sind.

Ferner kann die Polymeroberfläche in dem Teilbereich durch das Laserlicht so strukturiert werden, dass in die entstehenden Vertiefungen Fremdatome, vorzugsweise Luftmoleküle, eingelagert werden können, wodurch die Polymeroberfläche hydrophobisiert wird.

Alternativ oder zusätzlich zu dem Strukturieren der Polymeroberfläche wird die Polymeroberfläche gemäß einer bevorzugten Ausführungsform der vorliegenden Erfindung durch das Bestrahlen mit Laserlicht chemisch verändert, wodurch die Benetzbarkeit des bestrahlten Teilbereichs der Polymeroberfläche modifiziert wird.

Beispielsweise können während der Laserbehandlung durch Oxidationsvorgänge polare Gruppen an der Polymeroberfläche entstehen, die den hydrophilen Effekt verstärken.

Ein Beispiel für eine chemische Veränderung ist, dass bei Polyester durch Excimer LaserBestrahlung sowohl das Verhältnis von O zu C, als auch das Verhältnis von Ester- zu Carboxy-Gruppen geändert werden kann.

Eine weitere Möglichkeit der chemischen Veränderung besteht darin, dass durch geeignete Wahl der Laserparameter Bindungen des Polymers aufgetrennt werde, so dass auf der behandelten Oberfläche Fragmente des Polymers vorliegen, die die Benetzbarkeit der-Polymeroberfläche in dem bestrahlten Teilbereich modifizieren.

Gemäß einer bevorzugten Ausführungsform des erfindungsgemäßen Verfahrens wird mindestens ein Teilbereich der Polymeroberfläche durch das Bestrahlen mit Laserlicht hydrophobisiert. Dieser Teilbereich des mikrofluiden Systems dient beispielsweise dazu , die flüssige Probe in ihrem Fluss (zum Beispiel innerhalb einer Kapillare) zu verlangsamen oder abzustoppen oder die Benetzung des Teilbereichs durch die flüssige Probe (zum Beispiel bei der Probenaufgabe) zu verhindern. Durch das Hydrophobisieren einer bereitgestellten hydrophilen Polymeroberfläche in einem oder mehreren Teilbereichen kann ein Hydrophil-Hydrophob-Muster auf der Oberfläche erzeugt werden.

Gemäß dem erfindungsgemäßen Verfahren wird mindestens ein Teilbereich der Polymeroberfläche durch das Bestrahlen mit Laserlicht hydrophilisiert. Dieser Teilbereich des mikrofluiden Systems kann beispielsweise dazu dienen, die flüssige Probe in ihrem Fluss (zum Beispiel innerhalb einer Kapillare) zu beschleunigen oder die Benetzung des Teilbereichs durch die flüssige Probe (zum Beispiel bei der Probenaufgabe) zu erleichtern. Durch das Hydrophilisieren einer bereitgestellten hydrophoben Polymeroberfläche in einem oder mehreren Teilbereichen kann ein Hydrophil-Hydrophob-Muster auf der Oberfläche erzeugt werden.

Auf der Polymeroberfläche wird durch Bestrahlen mit Laserlicht verschiedener Parameter in verschiedenen Teilbereichen der Polymeroberfläche ein Hydrophil-Hydrophob-Muster erzeugt. Für ein solches Hydrophil-Hydrophob-Muster, bei dem sich hydrophile und hydrophobe Teilbereiche abwechseln, ist durch geeignete Wahl von Laser und Optik eine räumliche Auflösung bis in den µm-Bereich möglich. Das Hydrophil-Hydrophob-Muster wird somit ortsaufgelöst ohne die Verwendung von Masken und ohne die Abtragung von Material von der Polymeroberfläche erzeugt. Im Unterschied zu den bekannten Methoden, wie Beschichtungsverfahren, lassen sich mit dem erfindungsgemäßen Verfahren durch das Bestrahlen mit Laserlicht hydrophile und hydrophobe Bereiche in einem diagnostischen System gezielt in direkter Nachbarschaft realisieren. Dadurch lässt sich zum Beispiel die Fluidikkontrolle in einem Mikrokanalsystem einfach steuern.

Gemäß einer bevorzugten Ausführungsform der vorliegenden Erfindung wird ein Teilbereich der Polymeroberfläche mit dem Laserlicht zum Modifizieren der Benetzbarkeit bestrahlt, dessen Durchmesser kleiner 4 cm, bevorzugt kleiner 10 µm, besonders bevorzugt kleiner 1 mm ist.

Eine bevorzugte Ausführungsform der vorliegenden Erfindung besteht darin, dass mindestens ein Kapillarkanal in einer Polymeroberfläche eines Trägers bereitgestellt wird und die Polymeroberfläche im Inneren des Kapillarkanals durch Bestrahlen mit Laserlicht hydrophilisiert wird. Der Kapillarkanal dient zum Transport der flüssigen Probe aufgrund von Kapillarkräften (beispielsweise von der Probenaufgabezone eines Testelements zu einer Nachweiszone des Testelements).

Eine Aufgabezone ist in diesem Zusammenhang ein Bereich des mikrofluiden Systems, der dazu vorgesehen ist, eine flüssige Probe aufzunehmen, die in dem mikrofluiden System transportiert, gemischt, getrennt, mit Reagenzien kontaktiert und/oder anderweitig prozessiert wird. Eine Nachweiszone ist so gestaltet, dass sich dort bestimmte Bestandteile der flüssigen Probe oder ihrer Reaktion mit in der Nachweiszone vorhandenen Reagenzien nachweisen lassen. Ein Beispiel dafür ist eine Zone, in der eine Nachweisreaktion für Glukose in einer flüssigen Probe (zum Beispiel einer Blutprobe) und deren photometrische Auswertung stattfindet.

Durch das Bestrahlen der Polymeroberfläche im Inneren des Kapillarkanals mit Laserlicht wird der bestrahlte Bereich innerhalb des Kapillarkanals bei dieser Ausführungsvariante hydrophilisiert. Dadurch wird eine Wasser enthaltende flüssige Probe in dem hydrophilisierten Bereich (der vorzugsweise einen Kontaktwinkel α < 30° aufweist) schneller transportiert. Der Kapillarkanal weist vorzugsweise einen inneren Durchmesser < 3 mm (besonders bevorzugt 1,5 mm), eine Länge < 15 mm (besonders bevorzugt 7 mm) und eine Tiefe zwischen 0,04 und 0,1 mm (besonders bevorzugt 0,07 mm) auf. Die Umgebung des Mikrokanals bleibt bei der gezielten Bestrahlung der Polymeroberfläche im Inneren des Kapillarkanals unbehandelt. Sie kann jedoch auch gezielt durch Lasereinsatz hydrophobisiert werden. Das erfindungsgemäße Verfahren hat den Vorteil, dass sich durch Laserbehandlung nicht nur ebene Oberflächen behandeln lassen. Auch tiefe Strukturen (zum Beispiel Mikrokanäle) lassen sich durch den Laserstrahl erreichen, um die Oberfläche des Kanals durch das Laserlicht zu funktionalisieren.

Besonders bevorzugt wird ein aus einem Polymer bestehender Träger bereitgestellt, aus dem der mindestens eine Kapillarkanal ausgestanzt wird. Ein Träger aus Polymer-Vollmaterial hat den Vorteil, dass beim Ausstanzen keine sonstigen Schichten des Trägers beschädigt werden können. Im Stand der Technik werden häufig Träger mit mehreren Schichten verwendet, die zum Beispiel aus Klebebändern bestehen können. Ein Kapillarkanal wird dabei in den Träger so gestanzt, dass zum Beispiel eine hydrophobe Deckschicht im Bereich des Kanals ausgestanzt und eine darunter liegende hydrophile Schicht im Bereich des Kanals freigelegt wird. Dabei wird einerseits meist die hydrophile Schicht beschädigt oder zumindest mechanisch beansprucht, andererseits werden häufig störende Klebstoffschichten im Bereich des Kanals freigelegt. Das erfindungsgemäße Verfahren hat demgegenüber den Vorteil, dass sich die Produktionsprozesse vereinfachen. Zusätzliche Beschichtungsschritte oder der Einsatz von Hilfsmaterialien (zum Beispiel von Klebebändern) entfallen. Stanzprozesse zur Erzeugung des Mikrokanals (zum Beispiel Kiss-cut-Prozess) werden stark vereinfacht, da keine störenden Beschichtungen im Kanal vorhanden sind. Der Mikrokanal wird erst nachträglich mit dem Laser behandelt und dadurch hydrophilisiert.

Gemäß der vorliegenden Erfindung wird ein Testelement zur Bestimmung eines Analyten in einer Flüssigkeit hergestellt, umfassend einen Träger mit einer Polymeroberfläche, eine Aufgabezone für eine Probe der Flüssigkeit, eine Nachweiszone zur Bestimmung des Analyten und einen Kapillarkanal zum Transport der Probe von der Aufgabezone in die Nachweiszone, wobei die Polymeroberfläche in einem Bereich um die Aufgabezone durch Bestrahlen mit Laserlicht hydrophobisiert wird. Durch das Hydrophobisieren in dem Bereich um die Aufgabezone, in der sich die Öffnung des Kapillarkanals befindet und auf die ein Benutzer zum Beispiel Blut aufträgt, wird überschüssiges Blut entweder in den Kapillarkanal eingesaugt oder tropft von dem hydrophobisierten Bereich ab, so dass nur der Kapillarkanal des Testelements benetzt wird und eine Verschmutzung des Randbereichs des Testelements und eines das Testelement aufnehmenden Messgeräts vermieden werden. Die durch das Laserlicht hydrophobisierte Oberfläche kann beispielsweise eine Lotus-Effekt-Oberfläche sein.

### Beispiele

Erfindungsgemäß werden Teilbereiche der Oberflächen zweier Polyesterfolien (PET) mit Laserlicht bestrahlt. Unbehandeltes PET ist hydrophob und weist einen Kontaktwinkel von ca. 74° auf. Es werden zwei Polyestertypen (Melinex, 350 µm dick und Hostaphan, 12 µm dick) untersucht. Die folgenden drei Lasersysteme werden auf Melinex angewendet:
- Diodengepumpter Festkörperlaser im 4 f Modus: Wellenlänge 266 µm, Pulsweite 25 ns, Repititionsrate 30 kHz, Pulsenergie 10 µJ, Hatch distance (= Laserspot overlap entspricht dem mittleren Strukturabstand, beispielsweise von Tal zu Tal) 6 µm, Strahldurchmesser 18 µm
- Regenerativer Amplifier Picosekunden-Laser: Wellenlänge 1064 nm, Pulsweite 12 ps, Repititionsrate 50 kHz, Pulsenergie 30 µJ, Hatch distance 12 µm, Strahldurchmesser 20 µm und
- KrF Excimer Laser: Wellenlänge 248 nm, Pulsweite 30 ns, Repititionsrate 100 Hz, Pulsenergie 400 bis 500 mJ, Hatch distance 10 µm, Strahldurchmesser 10 µm.

Hostaphan wird ebenfalls mit dem oben genannten 4 f Laser mit 266 nm behandelt.

Zur Auswertung der Oberflächenmodifikationen durch das Laserlicht werden eine CCD-Kamera (Benetzungserkennung), ein Scanning Electron Microscope - SEM (Morphologie), ein Atomic Force Microscope - AFM (Rauhigkeit) und ein optisches Mikroskop eingesetzt. Die Kontaktwinkel in den Teilbereichen nach der Laserbehandlung betragen in allen Fällen <5°.

Typische Werte für die Tiefenprofile (Topographie) sind ca. 5 bis 6 µm für Melinex nach Bestrahlung mit dem 1064 nm Picosekunden-Laser.

Es ist weiterhin von chemischen Modifikationen der Oberflächen beispielsweise durch Oxidationen und Umlagerungen (bei Polyester von Ester- in Carboxy-Gruppen) auszugehen.

### Zeichnung

Anhand der Zeichnung wird die Erfindung nachstehend näher erläutert.

Es zieigen:
- Figur 1: in zwei verschiedenen Vergrößerungen einen entsprechend dem erfindungsgemäßen Verfahren mit Laserlicht bestrahlten Teilbereich einer Polymeroberfläche,
- Figuren 2A bis 2D: schematisch verschiedene Hydrophil-Hydrophob-Muster von mikrofluiden Systemen, die nach dem erfindungsgemäßen Verfahren herstellbar sind, und
- Figuren 3A und 3B: schematisch ein Testelement, das nach dem erfindungsgemäßen Verfahren herstellbar ist.

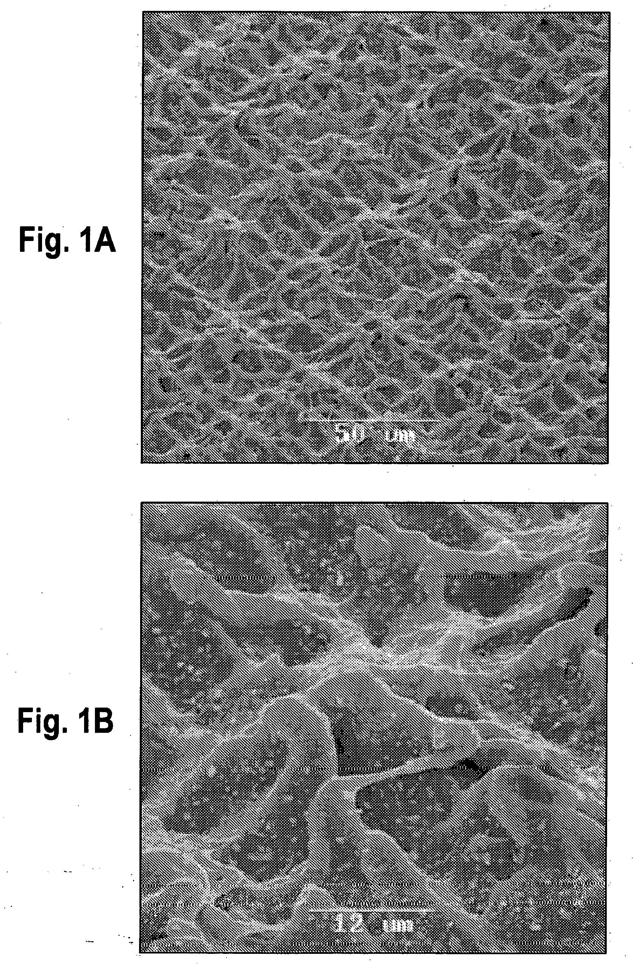

Figuren 1A und 1B zeigen zwei verschiedene Vergrößerungen einer Laser-strukturierten PET-Folie mit hydrophilen Eigenschaften. Durch die Bestrahlung des gezeigten Teilbereichs der Polymeroberfläche mit Laserlicht wurden gezielt runde, verschmolzene Mikrostrukturen erzeugt, die hydrophile Eigenschaften hervorrufen.

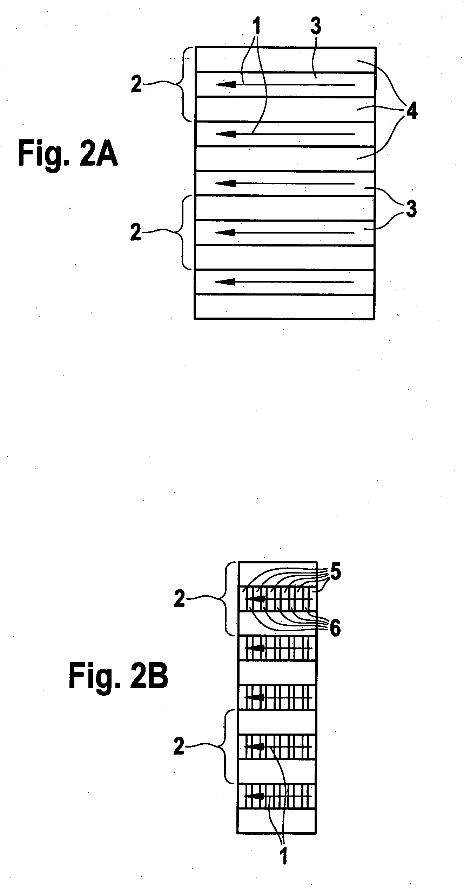

Figuren 2A bis 2D zeigen verschiedene erfindungsgemäße mikrofluide Systeme, die nach dem erfindungsgemäßen Verfahren herstellbar sind.

In Figur 2A sind fünf parallel zueinander angeordnete mikrofluide Systeme 2 dargestellt. Der Pfeil 1 steht jeweils für die Transportrichtung einer (nicht dargestellten) flüssigen Probe durch das mikrofluide System 2. Das mikrofluide System 2 weist jeweils ein Hydrophil-Hydrophob-Muster dergestalt auf, dass ein in Längsrichtung verlaufender hydrophiler Bereich 3 von zwei parallel dazu verlaufenden hydrophoben Bereichen 4 flankiert wird. Zur Herstellung dieses Hydrophil-Hydrophob-Musters wird zum Beispiel eine hydrophobe PET-Oberfläche in denjenigen Teilbereichen mit Laserlicht (zum Beispiel gemäß oben genannten Beispielen) bestrahlt, die zu den hydrophilen Bereichen 3 modifiziert werden. Die mikrofluiden Systeme 2 können zum Beispiel durch mittiges Durchtrennen (zum Beispiel Stanzen) der hydrophoben Bereiche 4 vereinzelt werden.

In Figur 2B sind ebenfalls fünf parallel zueinander angeordnete mikrofluide Systeme 2 dargestellt. In Transportrichtung 1, in der eine flüssige Probe durch das jeweilige mikrofluide System 2 aufgrund von Kapillarkräften transportiert wird, wechseln sich Zonen 5, die einen kleineren Kontaktwinkel α (vorzugsweise α < 30°) in Bezug auf Wasser aufweisen, mit Zonen 6, die einen größeren Kontaktwinkel α (vorzugsweise 30° < α < 90°) in Bezug auf Wasser aufweisen, ab. In diesem Zusammenhang bedeutet ein "kleinerer" Kontaktwinkel, dass dieser relativ zu dem "größeren" Kontaktwinkel einen kleineren Wert besitzt, wobei der kleinere Kontaktwinkel insbesondere zwischen 0° und 30° und der größere zwischen 30° und 90° liegen kann. Die Zonen, die kleinere Kontaktwinkel, vorzugsweise mit α < 30° bezüglich Wasser, aufweisen, sind schnellere Füllstrecken, auf die jeweils eine langsamere Füllstrecke mit größerem Kontaktwinkel α, vorzugsweise mit α > 30° folgt. Der Kontaktwinkel in den Zonen mit α > 30° bezüglich Wasser beträgt vorzugsweise 50° bis 85° für Wasser. Diese Zonen 5, 6 werden bei jedem mikrofluiden System 2 von zwei parallel zur Transportrichtung 1 verlaufenden hydrophoben Bereichen 4 flankiert. Die Zonen 5, 6 liegen vorzugsweise in einem Kapillarspalt.

Vorzugsweise umfassen die Zonen 5, 6, die in einer Kapillare in Transportrichtung aufeinanderfolgen, mindestens einer Reaktions-, Anreicherungs- oder Nachweiszone und mindestens eine Verzögerungszone, wobei in der Kapillare zweckmäßig zwischen je zwei sich unterscheidenden Zonen eine Verzögerungszone liegt. Eine Reaktionszone ist dabei eine Zone, in der die flüssige Probe mit dort platzierten Reagenzien reagiert. Dabei kann es sich zum Beispiel um Vorreaktionen, Entstörreaktionen oder Felder zur Reagenzientrennung handeln. In einer Anreicherungszone wird ein Bestandteil der flüssigen Probe angereichert. Eine Nachweiszone ist so gestaltet, dass sich dort bestimmte Bestandteile der flüssigen Probe oder ihre Reaktion mit den Reagenzien nachweisen lassen. Ein Beispiel dafür ist eine Zone, in der eine Nachweisreaktion für Glukose in einer Blutprobe und deren photometrische Auswertung stattfindet. In einer Verzögerungszone wird der Fluss der Probe (aufgrund eines größeren Kontaktwinkels) verzögert, so dass diese erst mit einer zeitlichen Verzögerung in die in Transportrichtung 1 auf eine Verzögerungszone nachfolgende Zone gelangt. In den Reaktions-, Anreicherungs- und Nachweiszonen verteilt sich die Probe (aufgrund eines kleineren Kontaktwinkels) schnell, damit sie mit den dort platzierten Reagenzien reagieren kann. In den Verzögerungszonen soll die Probe langsamer fließen, so dass sie eine gewisse Zeit benötigt, um sich aus der davor angeordneten Zone durch die jeweilige Verzögerungszone zu bewegen. Daher ist der Kontaktwinkel α, mit Wasser in den Reaktions-, Anreicherungs- oder Nachweiszonen kleiner (zur schnelleren Füllung) und in den Verzögerungszonen größer (zum "Zurückhalten" der Probe, also zur langsamen Füllung). Zwischen je zwei sich unterscheidenden Zonen liegt zweckmäßig (aber nicht zwingend) eine Verzögerungszone, um Reaktionen in den beiden anderen Zonen zu "trennen".

Das Hydrophil-Hydrophob-Muster der Zonen 5, 6 und der Bereiche 4 auf einer Polymeroberfläche wird erfindungsgemäß durch ein gezieltes Bestrahlen von Teilbereichen der Polymeroberfläche mit Laserlicht hergestellt, wodurch ein ortsaufgelöstes Modifizieren der Benetzbarkeit der Teilbereiche durch eine flüssige Probe erreicht wird.

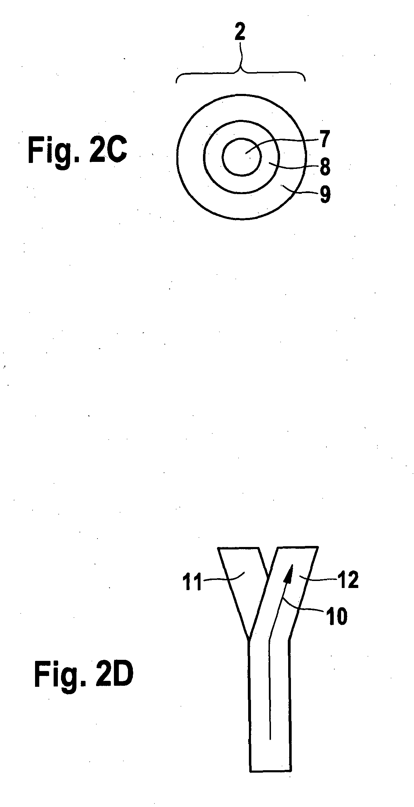

Figur 2C zeigt schematisch ein mikrofluides System 2, das als eine Hydrophob-Sperre dient. Das System weist einen hydrophilen inneren Bereich auf, der aus einem (7) oder zwei inneren hydrophilen Teilbereichen (7, 8) besteht und der von einem ringförmigen hydrophoben äußeren Teilbereich 8 bzw. 9 umgeben ist. Solche Hydrophob-Sperren sind wichtige Bestandteile eines diagnostischen Systems im Hinblick auf Hygiene-Aspekte und die Funktion des diagnostischen Systems. Die einzelnen Teilbereiche 7, 8, 9 werden erfindungsgemäß durch gezieltes Bestrahlen einer Polymeroberfläche mit Laserlicht hydrophilisiert oder hydrophobisiert.

Figur 2D zeigt schematisch ein weiteres durch das erfindungsgemäße Verfahren herstellbares mikrofluides System 2, bei dem sich die Flussrichtung 10 einer flüssigen Probe durch das Hydrophil-Hydrophob-Muster steuern lässt. Ein hydrophober Teilbereich 11 verhindert das Fließen einer flüssigen Probe in diesem Bereich der Polymeroberfläche. Die Probe fließt stattdessen entlang des hydrophilen Teilbereichs 12.

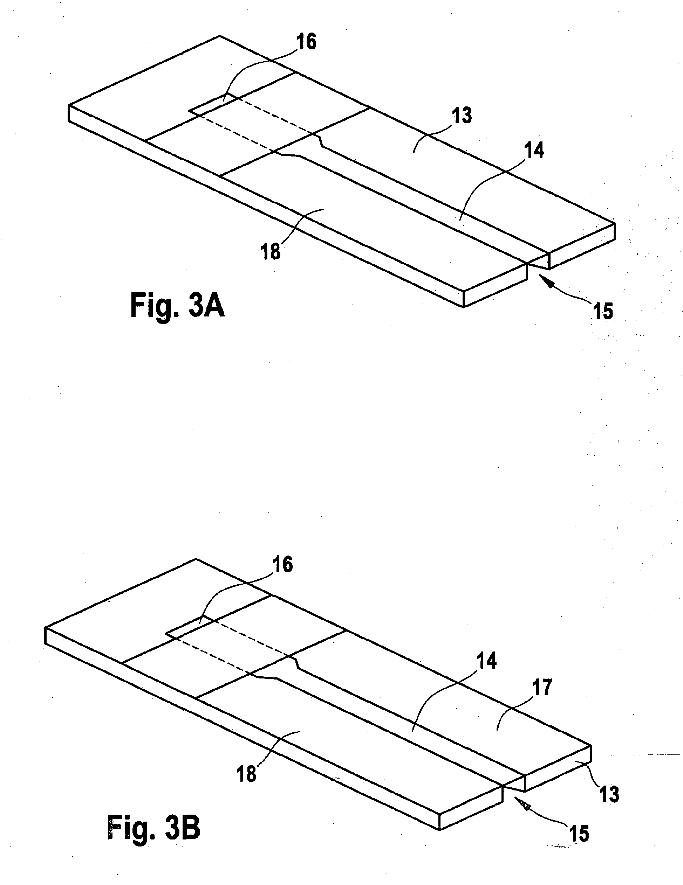

Figur 3A zeigt ein Testelement 13, das nach dem erfindungsgemäßen Verfahren hergestellt ist. Das Testelement 13 weist einen Träger 18 und einen in dem Träger 18 vorhandenen Kapillarkanal 14 auf, der von einer Aufgabezone 15 für eine flüssige Probe bis zu einer Nachweiszone 16 zur Bestimmung eines Analyten in der Probe verläuft. Der Träger 18 besteht aus einem Polymer, vorzugsweise aus PET. Die Polymeroberfläche im Inneren des Kapillarkanals 14 ist durch Bestrahlen mit Laserlicht hydrophilisiert worden.

Figur 3B zeigt ein entsprechend Figur 3A aufgebautes Testelement 13, bei dem zusätzlich die Polymeroberfläche 17 in der Umgebung des Kapillarkanals 14, insbesondere in dem Bereich um die Aufgabezone 15, durch Bestrahlen mit Laserlicht hydrophobisiert worden ist.

### Bezugszeichenliste

- 1: Transportrichtung
- 2: mikrofluides System
- 3: hydrophiler Bereich
- 4: hydrophobe Bereiche
- 5: Zonen mit kleinem α
- 6: Zonen mit großen α
- 7: erster hydrophiler innerer Bereich
- 8: zweiter hydrophiler innerer Bereich
- 9: hydrophober äußerer Bereich
- 10: Flussrichtung
- 11: hydrophober Teilbereich
- 12: hydrophiler Teilbereich
- 13: Testelement
- 14: Kapillarkanal
- 15: Aufgabezone
- 16: Nachweiszone
- 17: Polymeroberfläche in der Umgebung des Kapillarkanals
- 18: Träger

## Patentansprüche

1. Verfahren zur Herstellung eines mikrofluiden Systems auf einer Polymeroberfläche, wobei mindestens ein Teilbereich der Polymeroberfläche gezielt mit Laserlicht bestrahlt wird zum ortsaufgelösten Modifizieren der Benetzbarkeit des Teilbereichs der Polymeroberfläche durch eine flüssige Probe, wobei mindestens ein Teilbereich der Polymeroberfläche durch das Bestrahlen mit Laserlicht hydrophobisiert wird, wobei der Teilbereich dazu eingerichtet ist, die flüssige Probe in ihrem Fluss zu verlangsamen oder abzustoppen oder die Benetzung des Teilbereichs durch die flüssige Probe, insbesondere bei einer Probenaufgabe, zu verhindern, wobei ein Testelement (13) zur Bestimmung eines Analyten in einer Flüssigkeit hergestellt wird, umfassend einen Träger (18) mit einer Polymeroberfläche, einer Aufgabezone (15) für eine Probe der Flüssigkeit, einer Nachweiszone (16) zur Bestimmung des Analyten und einem Kapillarkanal (14) zum Transport der Probe von der Aufgabezone (15) in die Nachweiszone (16), wobei die Polymeroberfläche in einem Bereich um die Aufgabezone (15) durch Bestrahlen mit Laserlicht hydrophobisiert wird, **dadurch gekennzeichnet, dass** auf der Polymeroberfläche durch Bestrahlen mit Laserlicht verschiedener Parameter in verschiedenen Teilbereichen der Polymeroberfläche ein Hydrophil-Hydrophob-Muster erzeugt wird.

2. Verfahren gemäß Anspruch 1, **dadurch gekennzeichnet, dass** die Polymeroberfläche durch das Bestrahlen strukturiert wird.

3. Verfahren gemäß einem der Ansprüche 1 oder 2, **dadurch gekennzeichnet, dass** die Polymeroberfläche durch das Bestrahlen mit Laserlicht chemisch verändert wird.

4. Verfahren gemäß einem der Ansprüche 1 bis 3, **dadurch gekennzeichnet, dass** ein Teilbereich der Polymeroberfläche mit Laserlicht zum Modifizieren der Benetzbarkeit bestrahlt wird, dessen Durchmesser < 4 cm ist.

5. Verfahren gemäß einem der Ansprüche 1 bis 4, **dadurch gekennzeichnet, dass** mindestens ein Teilbereich der Polymeroberfläche durch das Bestrahlen mit Laserlicht hydrophilisiert wird.

6. Verfahren gemäß einem der Ansprüche 1 bis 5, **dadurch gekennzeichnet, dass** mindestens ein Kapillarkanal (14) in einem Träger (18) bereitgestellt wird und die Polymeroberfläche im Inneren des Kapillarkanals (14) durch Bestrahlen mit Laserlicht hydrophilisiert wird.

7. Verfahren gemäß Anspruch 6, **dadurch gekennzeichnet, dass** ein aus einem Polymer bestehender Träger (18) bereitgestellt wird, aus dem der mindestens eine Kapillarkanal (14) ausgestanzt wird.

## Claims

1. Method for producing a microfluidic system on a polymer surface, wherein at least one portion of the polymer surface is irradiated by laser light in a targeted fashion for the spatially-resolved modification of the wettability of the portion of the polymer surface by a liquid sample, wherein at least one portion of the polymer surface is hydrophobized by the irradiation with laser light, wherein the portion is designed to slow or stop the flow of the liquid sample or to prevent wetting of the portion by the liquid sample, particularly at the sample application, wherein a test element (13) for determining an analyte in a liquid is produced and comprises a carrier (18) with a polymer surface, an application zone (15) for a sample of the liquid, a detection zone (16) for determining the analyte and a capillary channel (14) for transporting the sample from the application zone (15) into the detection zone (16), wherein the polymer surface is hydrophobized in a region around the application zone (15) by irradiation with laser light, **characterized in that** a hydrophilic-hydrophobic pattern is generated on the polymer surface by irradiation with laser light with different parameters in different portions of the polymer surface.

2. Method according to Claim 1, **characterized in that** the polymer surface is structured by the irradiation.

3. Method according to one of Claims 1 and 2, **characterized in that** the polymer surface is changed chemically by the irradiation with laser light.

4. Method according to one of Claims 1 to 3, **characterized in that** a portion of the polymer surface is irradiated by laser light for modifying the wettability, the diameter of the portion being < 4 cm.

5. Method according to one of Claims 1 to 4, **characterized in that** at least one portion of the polymer surface is hydrophilized by the irradiation with laser light.

6. Method according to one of Claims 1 to 5, **characterized in that** at least one capillary channel (14) is provided in a carrier (18) and the polymer surface in the interior of the capillary channel (14) is hydrophilized by irradiation with laser light.

7. Method according to Claim 6, **characterized in that** provision is made for a carrier (18) composed of a polymer, with the at least one capillary channel (14) being stamped out of said carrier.

## Revendications

1. Procédé de fabrication d'un système microfluide sur une surface polymère, dans lequel au moins une partie de la surface polymère est irradiée de manière contrôlée par de la lumière laser pour modifier localement le mouillage de la partie de la surface polymère par un échantillon liquide,
au moins une partie de la surface polymère étant hydrophobisée par l'irradiation par de la lumière laser,
la partie étant conçue pour ralentir l'écoulement de l'échantillon liquide, le bloquer ou gêner le mouillage de la partie par l'échantillon liquide, en particulier à l'endroit de l'apport de l'échantillon,
l'élément de test (13) déterminant un analyte dans un liquide étant préparé et comprenant un support (18) doté d'une surface polymère, d'une zone d'apport (15) d'un échantillon de liquide, d'une zone de détection (16) qui détermine l'analyte et d'un canal capillaire (14) qui transporte l'échantillon depuis la zone d' apport (15) jusque dans la zone de détection (16), la surface polymère étant hydrophobisée par irradiation par de la lumière laser dans une partie qui entoure la zone d'apport (15),
**caractérisé en ce que**
un motif hydrophile-hydrophobe est formé sur la surface polymère par irradiation par de la lumière laser présentant différents paramètres dans différentes parties de la surface polymère.

2. Procédé selon la revendication 1, **caractérisé en ce que** la surface polymère est structurée par l'irradiation.

3. Procédé selon l'une des revendications 1 ou 2, **caractérisé en ce que** la surface polymère est modifiée chimiquement par l'irradiation par la lumière laser.

4. Procédé selon l'une des revendications 1 à 3, **caractérisé en ce qu'**une partie de la surface polymère dont le diamètre est < 4 cm est irradiée par de la lumière laser en vue de modifier sa mouillabilité.

5. Procédé selon l'une des revendications 1 à 4, **caractérisé en ce qu'**au moins une partie de la surface polymère est hydrophilisée par irradiation par la lumière laser.

6. Procédé selon l'une des revendications 1 à 5, **caractérisé en ce qu'**au moins un canal capillaire (14) est réalisé dans un support (18) et la surface polymère située à l'intérieur du canal capillaire (14) est hydrophilisée par irradiation par de la lumière laser.

7. Procédé selon la revendication 6, **caractérisé en ce qu'**un support (18) dans lequel le ou les canaux capillaires (14) sont estampés est réalisé en un polymère.
